Europäisches Patentamt

European Patent Office

Office européen des brevets

(19)

(11) **EP 1 258 228 A1**

(12) **DEMANDE DE BREVET EUROPEEN**

(43) Date de publication:
**20.11.2002 Bulletin 2002/47**

(51) Int Cl.⁷: **A61F 2/01**

(21) Numéro de dépôt: **02291131.7**

(22) Date de dépôt: **06.05.2002**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **18.05.2001 FR 0106587**

(71) Demandeur: **B. Braun Medical SAS**
**92100 Boulogne Billancourt (FR)**

(72) Inventeurs:
• **Nadal, Guy**
  **86000 Poitiers (FR)**
• **O'Connell, Paul, c/o B. BRAUN MEDICAL SAS**
  **92100 Boulogne Billancourt (FR)**

(74) Mandataire: **Lerner, François**
**Lerner & Associés,**
**5, rue Jules Lefèbvre**
**75009 Paris (FR)**

(54) **Appareil médical intraluminal comprenant deux parties séparables**

(57)    L'invention se rapporte à un appareil médical intraluminal adapté pour être disposé dans un conduit anatomique. L'appareil comprend une première partie (11) adaptée pour être retirée du conduit après y avoir été placée pendant une première période et une seconde partie (1) associée à un dispositif thérapeutique et liée à la première partie pendant la première période, par l'intermédiaire d'un moyen de retenue libérable (13). Pour assurer une séparation entre la première et la deuxième parties, le moyen de retenue libérable (13) présente une première liaison séparable qui se rompt si une première force de séparation est exercée entre la première et la deuxième parties à l'endroit du moyen de retenue libérable, alors que la seconde partie est dans un état de retenue, fixée au conduit (5) ou en appui contre une surface d'appui (53a, 81a) d'un élément complémentaire de l'appareil, cet élément complémentaire étant indépendant et monté mobile vis-à-vis des première et seconde parties, pour être retiré hors du conduit le moment venu.

FIG_3

EP 1 258 228 A1

## Description

L'invention concerne un appareil médical intraluminal adapté pour être introduit dans un conduit d'un corps vivant. Un filtre sanguin destiné à être disposé in situ dans un vaisseau est en particulier concerné, ce filtre pouvant être disposé dans la veine cave inférieure pour prévenir la migration d'emboles.

Dans certains cas, il est nécessaire, par exemple pour prévenir un risque important mais momentané d'embolie, de n'assurer une telle filtration sanguine que pendant un intervalle de temps limité, par exemple de deux à quatre semaines. Ensuite, le praticien peut décider que la filtration n'est plus utile.

Pour ce type de situation, on a déjà proposé un filtre "convertible" comme décrit dans US-A-5 383 887.

Dans d'autres situations, il s'avère utile de pouvoir retirer une partie de l'appareillage médical qui a été, pendant un moment, introduit dans le conduit anatomique concerné.

Tel est le cas dans US-A-5 108 407 où un enroulement (coil) est à placer, seul, dans une cavité d'anévrisme, ou dans EP-A-0 925 763 où une structure expansible radialement à l'axe du conduit doit y être larguée, seule, pour l'occlure. Le conduit peut être un vaisseau, un conduit biliaire, l'oesophage, ou autres.

Ainsi, est déjà connu un appareillage intraluminal comprenant :

- une première partie adaptée pour être retirée du conduit après y avoir été placée pendant une première période,
- et une seconde partie liée à la première partie pendant la première période, par l'intermédiaire d'un moyen de retenue libérable, cette seconde partie étant donc placée avec la première partie et le moyen de retenue libérable dans le conduit, pendant la première période, cette seconde partie étant constituée par, ou comprenant, un dispositif thérapeutique adapté pour demeurer dans le conduit après la première période.

Le problème posé ici concerne la séparation entre elles des première et seconde parties par un moyen mécanique fiable et sûr pour le patient et qui soit réalisable compte tenu de la taille des pièces concernées, vu les conditions d'intervention.

La solution proposée dans l'invention prévoit que, pour assurer une séparation entre la première et la deuxième parties et permettre à la deuxième partie de demeurer dans le conduit à l'issue de la première période, le moyen de retenue libérable présente une première liaison séparable qui se rompt si une première force de séparation est exercée entre la première et la deuxième parties à l'endroit du moyen de retenue libérable, alors que la seconde partie est dans un état de retenue, fixée au conduit ou en appui contre une surface d'appui d'un élément complémentaire de l'appareil, cet élément complémentaire étant indépendant et monté mobile vis-à-vis des première et seconde parties pour être retiré hors du conduit le moment venu.

Dans ce contexte, l'invention s'est en outre attachée à assurer à la fois un maintien sûr entre les parties pendant leur coopération et une séparation qui puisse s'effectuer sans (trop) d'à coups et avec une réaction très limitée à la séparation.

C'est pourquoi, une caractéristique complémentaire de l'invention conseille que, pour constituer ladite première liaison séparable, au moins un élément allongé dur est associé au moyen de retenue libérable et est lié de façon permanente à la première ou à la seconde partie, ainsi qu'un bloc de matériau plus malléable que la matière de l'élément allongé dur, l'élément allongé dur étant noyé à l'intérieur du bloc de matériau malléable pendant la première période et en étant séparé à force lorsque ladite première force de séparation est exercée entre la première et la seconde parties.

Avantageusement, la matière du bloc de matériau malléable est, ou comprend, une silicone et l'élément allongé dur est en un matériau parmi un métal, un alliage métallique (en particulier, un alliage à mémoire de forme thermique), voire éventuellement un plastique biocompatible plus dur que la silicone.

Pour accroître la sécurité du maintien pendant la première période, sans interférer avec une séparation "en douceur", il est par ailleurs conseillé que le(les) élément(s) allongé(s) dur(s) se termine(nt) par une zone de retenue renforcée présentant une section élargie ou une partie terminale courbée.

La sécurité du patient étant primordiale, il est apparu raisonnable de malgré tout tenir compte d'une complication pouvant survenir au moment de la séparation souhaitée.

Aussi, une autre caractéristique de l'invention conseille-t-elle que l'élément de retenue libérable présente en outre une seconde liaison séparable existant, à l'issue de ladite première période, entre l'élément de retenue libérable et des moyens d'accouplement retirables du conduit à l'issue de la première période et adaptés pour se séparer de l'élément de retenue si une seconde force prédéterminée de séparation est exercée entre eux, cette seconde force étant supérieure à la première, en valeur nominale.

Pour conjuguer la sécurité du patient avec une exigence d'efforts minimum gage d'efficacité, on conseille que $2\,N \leq F2 \leq 3{,}5\,N$ et $3\,N \leq F3 \leq 4\,N$.

Comme déjà noté, un domaine d'application privilégié de l'invention concerne les filtres sanguins.

Aussi, l'invention s'est-elle en particulier attachée à prendre en compte les problèmes posés par un dispositif de filtration comprenant un filtre sanguin convertible, dans le vaisseau où il a été implanté, en une structure perméable au sang et demeurant implanté dans ce vaisseau, bien qu'il n'assure alors plus de fonction de filtration.

Tel que décrit dans US-A-5 383 887, un tel filtre comprend :

- une pluralité d'éléments filtrants intraluminaux, les éléments étant agencés pour être disposés dans une configuration de filtration dans la lumière du vaisseau, ces éléments filtrants présentant une zone à l'endroit de laquelle ils sont radialement rapprochés les uns des autres dans cette configuration de filtration,
- un élément de jonction relié à la pluralité des éléments filtrants et qui, au moins à la libération de l'élément de retenue, joint entre eux les éléments filtrants alors disposés dans une configuration ouverte où ces éléments sont radialement écartés les uns des autres, l'élément de retenue libérable joignant les éléments filtrants entre eux dans la configuration de filtration.

D'autres caractéristiques concernant spécifiquement un tel filtre apparaissent dans les revendications annexées et ont pour but :

- d'assurer une sécurité tant pour obtenir l'ouverture du dispositif filtrant que pour maintenir celui-ci dans sa configuration de filtration (tant que la libération du moyen libérable n'a pas été décidée et entreprise),
- d'éviter alors une ouverture défectueuse, uniquement partielle, du filtre.

Un autre problème concerne la référence pour définir les valeurs des forces de retenue/séparation à assurer entre les pièces du dispositif de filtration lequel comprend tant le filtre que les moyens pour séparer l'élément de retenue du filtre.

A cet égard, il a été décidé de définir les forces de séparation entre ces différents éléments en partant de la force "d'accouplement" (c'est-à-dire de retenue) du filtre vis-à-vis de la paroi interne du vaisseau, à l'issue d'une certaine période d'implantation (typiquement au moins 15 jours).

Ainsi, après différents essais menés, et en tirant partie d'expériences antérieures, il a été considéré que les caractéristiques suivantes étaient favorables (lorsque ladite seconde force devient inférieure à la première dans des conditions particulières) :

- la séparation des moyens d'accouplement vis-à-vis de l'élément de retenue s'opère par déformation d'une zone des moyens d'accouplement, sous l'effet de la seconde force exercée, ou
- la séparation des moyens d'accouplement vis-à-vis de l'élément de retenue s'opère par rupture mécanique d'une partie des moyens d'accouplement liée au filtre, sous l'effet de la seconde force exercée, ou
- la séparation des moyens d'accouplement vis-à-vis de l'élément de retenue s'opère par passage à force d'une partie des moyens d'accouplement à travers un orifice calibré dans lequel ces moyens d'accouplement sont retenus jusqu'à ce que ladite seconde force soit exercée.

Une description plus détaillée de l'invention va maintenant être fournie en référence aux dessins d'accompagnement dans lesquels :

- la figure 1 montre un filtre sanguin conforme à l'invention dans sa configuration de filtration,
- la figure 2 montre le filtre de la figure 1 dans sa configuration ouverte, après retrait de l'élément de retenue libérable,
- la figure 3 montre une vue agrandie du détail repéré III sur la figure 1,
- la figure 4 montre différentes formes possibles d'une partie extrême des pattes du filtre,
- les figures 5 et 6 montrent deux variantes du détail III de la figure 1,
- les figures 7, 8 et 9 montrent trois situations correspondant à la libération d'un dispositif radialement expansible dans un conduit, dans le cas où la première liaison séparable se libère correctement (figure 8) et dans le cas où seule la seconde liaison se libère (figure 9), avec sur chaque figure une représentation avec arrachement partiel à l'endroit des branches du dispositif expansible qui doit être libéré dans le conduit.
- la figure 10 montre le principe d'une implantation d'un enroulement filamentaire pour anévrisme,
- la figure 11 détaille cet élément filamentaire et son moyen de retenue libérable,
- la figure 12 est une vue agrandie du détail XII de la figure 11 qui montre également le poussoir et le système à lasso qui permettent la libération de l'enroulement filamentaire, lequel est illustré sur la figure 13 dans la situation où la première liaison séparable s'est correctement libérée.

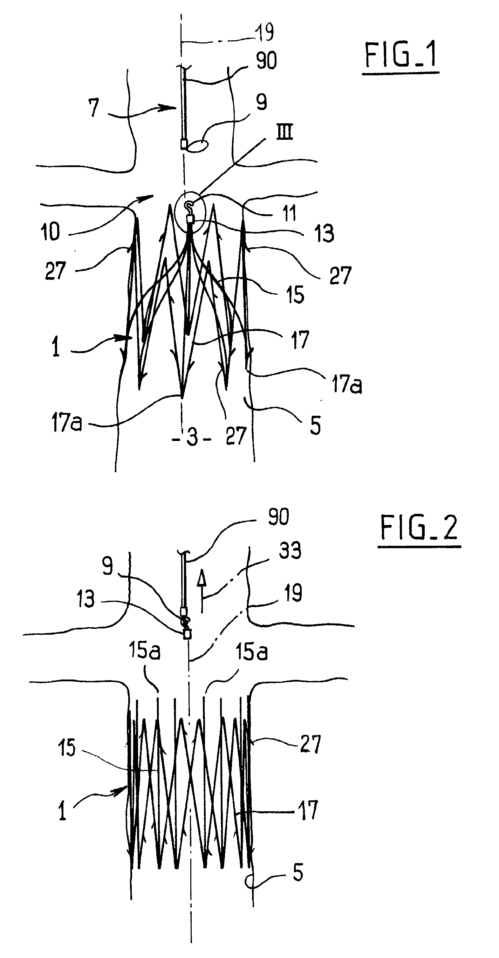

Sur la figure 1 tout d'abord, on voit un filtre sanguin 1 dans sa configuration de filtration. Le filtre 1 est implanté dans la lumière 3 d'un vaisseau sanguin 5, avec un axe 19 de préférence confondu avec celui du vaisseau. Ce vaisseau est ici la veine cave inférieure.

Considéré dans son ensemble, le dispositif de filtration complet 10 comprend en outre un système de libération 7 terminé par un dispositif d'accouplement 9 adapté pour agir sur le moyen d'accouplement 11 du filtre 1 qui, lorsqu'il est attrapé par le moyen complémentaire 9 libère l'élément de retenue libérable 13 auquel il est lié, permettant ainsi de faire passer les pattes 15 du filtre de leur configuration de filtration de la figure 1 à leur configuration ouverte de la figure 2 où le filtre 1 se présente comme un stent.

Comme illustré sur les figures 1 et 2, le filtre 1 peut comprendre un fil unique (ou une succession de fils réunis bout à bout, par exemple par soudage) disposé(s) suivant une configuration en zigzag fermée sur elle-même pour définir ainsi une structure tubulaire 17, en absence de contrainte exercée radialement à l'axe du tube (axe 19).

Dans cette configuration "radialement déployée", cette structure tubulaire en zigzag, ou ondulée, présente une succession de tronçons sensiblement en "V", successivement dans un sens et puis dans l'autre, sur le périmètre du tube. Pour la retenue du filtre dans le vaisseau, la structure 17 (et/ou les pattes 15) est pourvue de moyens "d'accrochage" à la paroi de ce vaisseau. Il peut s'agir de crochets 27.

Pour la clarté de la présentation, on appellera "extrémité proximale" l'extrémité du filtre 1 où est situé l'élément de retenue libérable 13, et "extrémité distale" l'extrémité opposée. Les crochets 27 peuvent être situés vers les deux extrémités.

Du côté de cette extrémité distale, de certains au moins des apex 17a de la structure 17 en zigzag partent les pattes de filtration 15 fixées à cette structure.

Sur l'illustration, les pattes (ou éléments filtrants) 15 se présentent comme des éléments allongés flexibles tels que des filaments rectilignes, présentant une extrémité libre 15a, à l'extrémité proximale du filtre (voir figure 2).

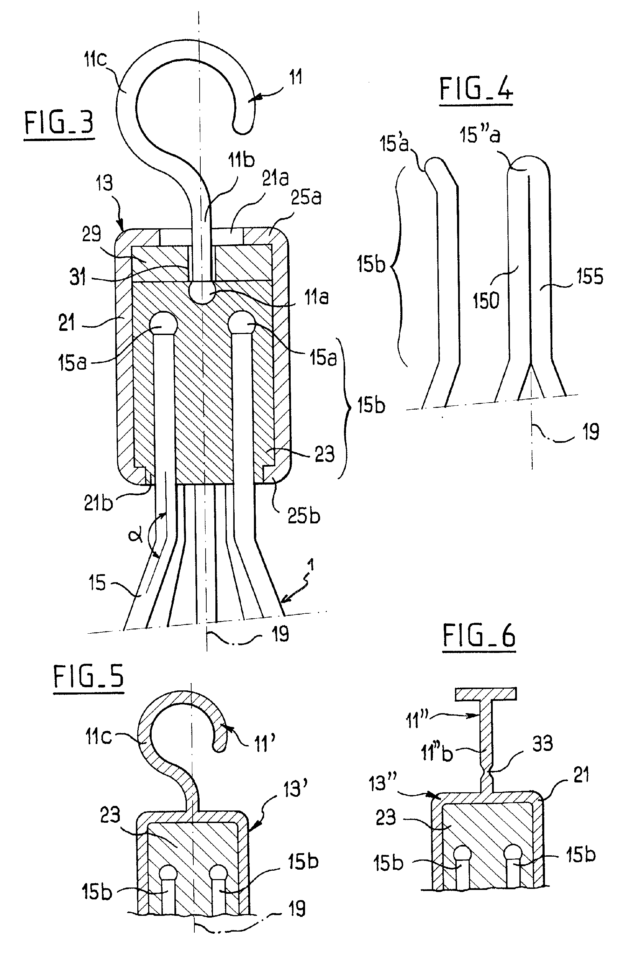

Dans la "configuration de filtration", lesdites pattes 15 sont, sous contrainte, rapprochées les unes des autres radialement, à leur extrémité proximale 15a, pour être réunies à l'intérieur de l'élément de retenue libérable 13, comme on peut le voir plus clairement sur la figure 3.

Sur la figure 2, on peut en outre constater qu'en l'absence de contrainte radiale des pattes par l'élément 13, ces pattes se présentent effectivement à la manière de fils essentiellement parallèles à l'axe 19 du filtre et parallèles donc à la génératrice du tube cylindrique auquel appartiennent les filaments en zigzag de la structure 17, dans l'état radialement non contraint du filtre 1.

Ainsi conçu, le filtre 1 présente donc un élément de jonction 17 qui, notamment à la libération de l'élément de retenue 13, joint entre elles les pattes 15, lesquelles du fait du retrait de l'élément 13 ont pu alors se déployer radialement à leur extrémité 15a, jusqu'à adopter la configuration ouverte à la manière d'un stent où elles sont écartées radialement les unes des autres, comme montré sur la figure 2. Les pattes 15 peuvent être élastiquement déformables pour passer d'une configuration à l'autre, ou être réalisées en un matériau à mémoire de forme thermique (Nitinol®, par exemple).

Bien entendu, d'autres structures que celle 17 illustrée, par exemple un ou plusieurs anneaux de cerclage radialement déformables, auraient pu être retenues pour assurer la jonction entre eux des éléments filtrants dans ladite configuration ouverte, et pour permettre d'assurer une cohésion structurelle au filtre, dans cette configuration.

Quant à l'élément de retenue libérable 13, il comprend un capuchon rigide 21 enserrant un bloc de matériau 23 à l'intérieur duquel la partie extrême des pattes 15 proche de l'extrémité 15a est noyée.

Sur la même figure, on a référencé 15b la zone des éléments filtrants 15 à l'endroit de laquelle chacun d'eux est noyé à l'intérieur du bloc de retenue 23.

Ce bloc est de préférence en matériau plus malléable que celui du capuchon 21. Il peut s'agir d'une silicone par opposition à un métal (par exemple en acier inoxydable ou encore une matière plastique dure utilisable pour le capuchon 21).

A ses extrémités axiales opposées, le capuchon 21 présente deux ouvertures, respectivement proximale 21a et distale 21b. Chacune d'elles est limitée périmétriquement par un épaulement, respectivement 25a et 25b.

A travers lesdites ouvertures, passent respectivement la base 11b du crochet 11 et chacune des pattes 15, alors dans leur configuration de filtration.

La matière relativement malléable du bloc 23 va permettre de doser avec une sécurité et une fiabilité importante les forces de séparation à l'endroit de l'élément de retenue libérable 13.

En outre, l'utilisation de cette matière malléable permet la création du bloc 23 par coulée.

Pour le calibrage des forces de séparation pattes /bloc et crochet/rondelle, le crochet 11 et/ou l'extrémité 15a de la zone de liaison 15b des pattes sont avantageusement pourvus d'une forme adaptée pouvant présenter une section plus importante que celle du reste de l'élément (11 ou 15).

Ainsi, le moyen d'accouplement 11 ou les pattes 15 pourront présenter une tête d'extrémité de plus gros volume, telle que 11a, ou 15a, avec une conformation parallélépipédique ou sphérique, par exemple, tandis qu'une autre solution pourrait être de courber cette extrémité distale, comme dans l'exemple 15'a, ou encore, pour les pattes 15, de les réunir deux à deux, à leur extrémité distale 15"a, par une forme en épingle à cheveux, avec deux tronçons 150,155 parallèles et plaqués l'un contre l'autre, parallèlement à l'axe 19 (voir figure 4).

On aura également noté au vu des figures 3 et 4 que, dans la zone 15b, les pattes 15 et la base 11a du crochet s'étendent (essentiellement) parallèlement à l'axe 19, de manière à ne pas constituer un obstacle défavorable à l'ouverture du filtre, lors du retrait de l'élément 13. Par contre, peu après avoir débouché de l'élément 13, les pattes s'évasent rapidement suivant un angle obtus $\alpha$ qui peut être commun à toutes les pattes, tel que $\alpha$ typiquement compris entre 140° et 170°.

La section de ces zones renflées 11a, 15a, ..., est donc fonction de la force de retenue à atteindre. Pour le moyen 11, c'est plus exactement le rapport de section entre la base 11b (ou l'extrémité renflée) et l'orifice calibré 31 de la rondelle 29, ainsi que le choix des matériaux, qui sont à considérer.

Pour ce qui concerne le moyen d'accouplement 11, une autre solution aurait pu consister à utiliser une rondelle 29 en une matière quasiment non malléable (métal par exemple) et à enfoncer à force la base 11b (alors dépourvue d'extrémité renflée) du crochet à l'intérieur d'un trou calibré présentant un diamètre uniquement supérieur de quelques dixièmes de millimètre au diamètre de la base 11b.

Dans chaque cas, c'est toutefois bien l'obstacle (constitué par l'orifice calibré) au libre passage du moyen 11 qui va définir la force de retenue de ce moyen 11 par rapport à l'élément 13.

Dans l'exemple de la figure 3, la rondelle 29 qui est donc interposée, à l'intérieur du capuchon 21, entre son épaulement proximal 25a et le bloc 23, est en une matière plastique plus rigide que celle de ce bloc 23, par exemple en POM (Polyoxyméthylène).

La solution alternative de la figure 5 montre que le moyen d'accouplement 11' pourrait être solidaire de, ou fixé intimement à, l'élément 13' (par exemple soudé) et présenter une capacité de déformation de sa zone courbée en forme d'anse 11c qui pourrait s'ouvrir en se déployant le long de l'axe 19 afin de laisser alors échapper le collet 9 si une certaine force de traction sur le crochet est atteinte.

Comme illustré sur la figure 6, une autre solution aurait également pu être que la base 11"b du moyen 11" présente un étranglement 33, juste après la liaison (par exemple soudée) entre le capuchon 21 de l'élément 13" et le moyen 11" qui présente ici une forme en "T".

On remarque que dans le cas des figures 5 et 6, on s'est dispensé de la rondelle de sécurité, le bloc 23 en matériau malléable occupant tout le volume intérieur libre dudit capuchon.

Toujours concernant ces "forces de retenue", appelons F1 la force de retenue entre le filtre (en particulier sa structure tubulaire filamentaire 17) et la paroi interne du vaisseau 5, appelons F2 la force de séparation entre la zone 15b des pattes 15 et l'élément 13, ou 13', ou 13" (en l'espèce, en particulier son bloc interne 23) et appelons F3 la force de séparation entre le moyen d'accrochage ou d'accouplement 11, et ledit élément de retenue libérable 13 (F3 pourrait également être la force d'ouverture de l'anse 11c ou la force de rupture de la zone de striction 33).

Compte tenu des objectifs de sécurité précités, il est conseillé que, en valeur nominale :

$$F2 < F3 < F1$$

Les valeurs conseillées sont les suivantes (en Newtons) :

$$3,5\,N \leq F1 \leq 6\,N$$

$$3\,N \leq F3 \leq 4\,N$$

$$2\,N \leq F2 \leq 3,5\,N$$

Ainsi, F1, correspond à la force de retenue assurée ici par les crochets 27, augmentée éventuellement de la force supplémentaire de retenue obtenue par le développement cellulaire qui s'opère autour au moins des zones du filtre en contact avec la paroi du vaisseau, du fait de son implantation.

En prenant pour exemple la solution illustrée figure 3, le fonctionnement d'ensemble du système peut être le suivant :

Supposons d'abord que le système de libération 7 comprenne un collet 9, typiquement appelé "lasso" en français ou "goose neck snare" en anglais. Le modèle "Amplatz goose neck"® de la société MICROVENA serait à cet égard tout à fait approprié, qu'il s'agisse d'une version "Snare" ou "Microsnare".

Sa boucle en collet 9 qui est radio-opaque s'étend à 90° de l'armature 90 qui la maintient et qui permet, par une manoeuvre de traction, de serrer plus ou moins le collet 9. La boucle en collet et l'armature longitudinale flexible 90 peuvent être en matériau mémoire de forme (Nitinol®, en particulier).

Pour ce qui concerne l'implantation, le filtre 1 a pu être mis en place par la méthode classique dite "de Seldinger", à l'intérieur d'une gaine, le déplacement axial relatif entre la gaine et la tige-poussoir interne engagée dans la gaine assurant la sortie du filtre hors de la gaine. Alors qu'il était radialement contraint à l'intérieur de la gaine, le filtre, une fois sorti, s'expanse radialement de manière que sa structure tubulaire 17 se plaque contre la paroi du vaisseau 5, en centrant le filtre. Les pattes 15 sont alors dans leur "configuration de filtration", en étant rapprochées les unes des autres en 15b, à l'intérieur de l'élément de retenue libérable 13.

Une fois la période souhaitée de filtration terminée, typiquement environ quatre semaines, le système de libération 7 est avancé par la même voie d'accès intraluminal que celle utilisée pour l'implantation du filtre, et amené jusqu'à l'extrémité proximale du filtre 1. Le collet 9 vient alors agripper le moyen d'accouplement 11.

Dans un cas normal, une traction appropriée sur le collet 9 depuis l'extérieur du corps du patient, suivi d'une traction sur l'armature 90 (qui ressort de ce corps) tire l'élément 13 dans le sens de la flèche 33 de la figure 2 et doit entraîner la séparation d'avec le filtre 1, en toute sécurité, puisque F2 < F1.

Dans cette hypothèse, l'ensemble 7/13 est entièrement et définitivement retiré du corps du patient et le filtre 1 s'ouvre radialement comme sur la figure 2 pour prendre définitivement une forme comme un stent, avec ses pattes 15 de préférence plaquées le long de la paroi interne du vaisseau 5, parallèlement à l'axe 19.

Si par contre la tête du filtre est par exemple incorporée dans un thrombus, c'est alors (dans l'exemple de la figure 3) entre le crochet 11 et la rondelle 29 que va s'effectuer la séparation, lorsque le collet 9 va tirer sur le crochet, ceci évitant d'exercer une traction exagérée sur la paroi du vaisseau et évitant également toute ouverture partielle de la tête du filtre.

Dans cette hypothèse, lorsque (la force F2 ayant augmenté du fait du thrombus, jusqu'à F2 > F3), la force F3 est atteinte, la base 11b du crochet passe en face à travers l'orifice calibré 31 et le filtre 1 reste avec ses pattes 15 dans sa configuration de filtration (figure 1), c'est-à-dire que le filtre ne s'ouvre pas. Le crochet est par contre retiré avec le collet.

A noter que dans le cas de la figure 5, le "moyen d'accouplement" est en réalité constitué par le système de libération, puisque, dans ce cas, l'élément 11' demeure solidaire du capuchon 21 lorsque, sous l'effort F3, cet élément 11' se déforme et laisse s'échapper le collet qui glisse.

Concernant l'ensemble 7 - 11, d'autres réalisations sont possibles, comme dans US 6 146 404 par exemple (cf. ensemble 164 - 220).

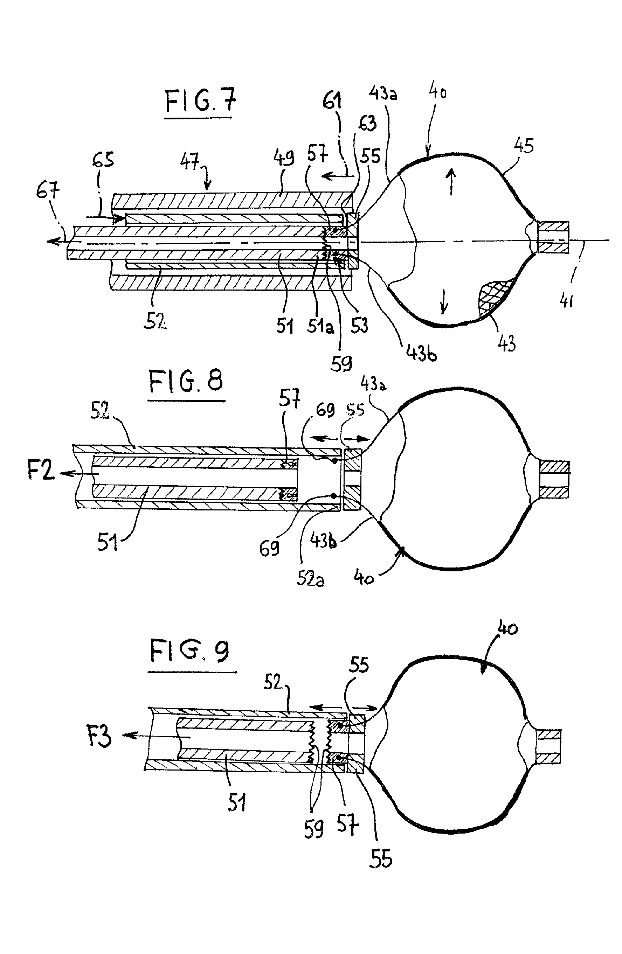

Sur les figures 7, 8 et 9, on voit l'application de l'invention à un autre dispositif intraluminal 40 consistant en une structure radialement expansible par rapport à l'axe général de l'appareil, 41, qui est également typiquement l'axe du conduit anatomique à l'endroit de la zone d'implantation de sa structure 40.

La structure 40 peut être utilisée comme un dispositif thérapeutique de protection distale servant soit à occlure, soit à filtrer le sang, dans un vaisseau, par exemple en aval d'une zone d'intervention où un élargisseur (stent) a été posé.

Le dispositif 40 est typiquement auto-expansible radialement (voir les flèches sur la figure 7) et peut être constitué avec une armature de fils tressés 43, tels en particulier que des fils métalliques élastiquement déformable.

Si le dispositif 40 doit être occlusif, la structure filamentaire 43 pourra être recouverte d'une membrane étanche aux liquides telle qu'un film en polyuréthanne, 45.

Pour plus de détails concernant la constitution structurelle du dispositif 40, on peut se reporter à EP-A-0 947 168, colonne 4, ligne 5 à colonne 6, ligne 4.

Dans le cas présent, plutôt qu'un dispositif de protection distal comme déjà évoqué, il paraît plus probable que le dispositif 40 ici illustré puisse être utilisé en embolothérapie, comme évoqué en colonne 1, lignes 11 à 22 de EP-A-0 947 168.

En particulier dans une telle application, le dispositif 40 doit rester implanté seul, indépendamment de ses moyens d'introduction endoluminale 47.

Les moyens 47 comprennent une gaine extérieure 49 à l'intérieur de laquelle le dispositif 40 est monté coulissant (dans un état radialement restreint, non représenté) à l'extrémité distale d'un guide 51 pouvant constituer en un tube à l'intérieur duquel peut passer un fil-guide ("guide wire" ou guide J, non représenté) s'étendant à l'intérieur de la gaine 49 jusqu'à l'extérieur du corps du patient et autour duquel glisse également librement, à l'intérieur de cette gaine, un poussoir tubulaire 52 (appelé également élément complémentaire axialement mobile).

A son extrémité distale (la plus profondément introduite dans le corps du patient) 51a, le tube de guidage 51 est prolongé par le moyen de retenue libérable 53 à l'endroit duquel il va pouvoir y avoir séparation entre le dispositif thérapeutique 40 (appelé dans les revendications " seconde partie") et les autres éléments précités (regroupés sous l'appellation "première partie" dans les revendications), ainsi qu'on peut le voir sur les figures 8 et 9 où la séparation du dispositif 40 est déjà intervenue.

Dans la solution des figures 7 à 9, le moyen de retenue libérable 53 comprend deux anneaux axialement accolés 55, 57, à travers lesquels passent plusieurs éléments allongés durs constitués ici par certains des fils métalliques, tels que 43a, 43b, du tressage 43

Comme dans le cas de la figure 3, l'anneau 57 est constitué en un matériau malléable (typiquement une silicone) pour permettre une séparation des fils, ou branches, 43a, 43b, vis-à-vis de cet anneau 57, le moment venu, ainsi que l'illustre la figure 8.

Typiquement, les branches 43a, 43b, seront en métal ou en un alliage métallique tel qu'un alliage à mémoire de forme thermique (Nitinol® en particulier).

L'anneau 55 peut être en un matériau plus dur que celui de l'anneau 57, par exemple en POM.

Par sécurité, une deuxième liaison séparable peut avoir été prévue à l'endroit de l'élément de retenue libérable 53, constituée (dans l'exemple illustré) par une ligne d'affaiblissement 59 située à la limite proximale de l'anneau 57, là où cet anneau est lié au guide tubulaire 51.

Eventuellement, l'anneau 57 et le guide 51 peuvent être réalisés en une seule pièce susceptible d'être mécaniquement affaiblie par exemple par une fente à l'endroit de la ligne 59. Deux matières différentes sont malgré tout peut-être préférable, de manière que l'embout annulaire 57 puisse être en silicone.

Le fonctionnement opérationnel peut se dérouler comme suit, le dispositif thérapeutique 40 pouvant être déjà sorti de sa gaine extérieure protectrice 47.

Dans l'exemple illustré, le dispositif 40 s'est même déjà radialement auto-expansé dans le conduit, après qu'on ait tiré vers l'arrière la gaine, dans le sens de la flèche 61.

A ce moment, le praticien applique la surface d'appui 52a du poussoir 52 contre l'anneau 55, à l'endroit de l'épaulement 63 que celui-ci présente vis-à-vis de l'anneau 57. Une telle poussée axiale dans le sens de la flèche 65, conjuguée avec une traction arrière dans le sens de la flèche 67 sur le guide 51 doit, après un calibrage approprié, provoquer la sortie des branches 43a, 43b, hors de la matière malléable de l'anneau 57, ainsi que l'illustre la figure 8 (effort F2). A noter la présence d'une tête élargie 69 à l'extrémité des branches 43a, 43b, pour le même effet que celui déjà cité pour la figure 3. A ce moment, la gaine 47 a pu être complètement retirée du corps du patient, ou non.

Quoi qu'il en soit, une fois le dispositif 40 ainsi libéré de son dispositif d'introduction, tant le guide 51 que le poussoir 52 peuvent être retirés du corps du patient, seul le dispositif 40 étant maintenu en place dans le conduit.

Si, malgré tout, l'effet de séparation n'est pas atteint, une seconde possibilité demeure et le praticien peut continuer à exercer un effort supplémentaire sur le poussoir 52 vis-à-vis de l'anneau 55, tout en tirant axialement sur le guide 51, jusqu'à atteindre l'effort F3, avec F3 > F2, ainsi que le montre la figure 9.

Cet effort F3 atteint, il doit y avoir rupture mécanique de la liaison en 59, comme le montre la figure 9, et donc séparation du guide 51 et de l'ensemble constitué par le dispositif 40 et les anneaux 55, 57.

Le dispositif 40 ainsi complété est alors laissé en place dans le conduit, tandis que les autres pièces sont retirées hors du corps du patient.

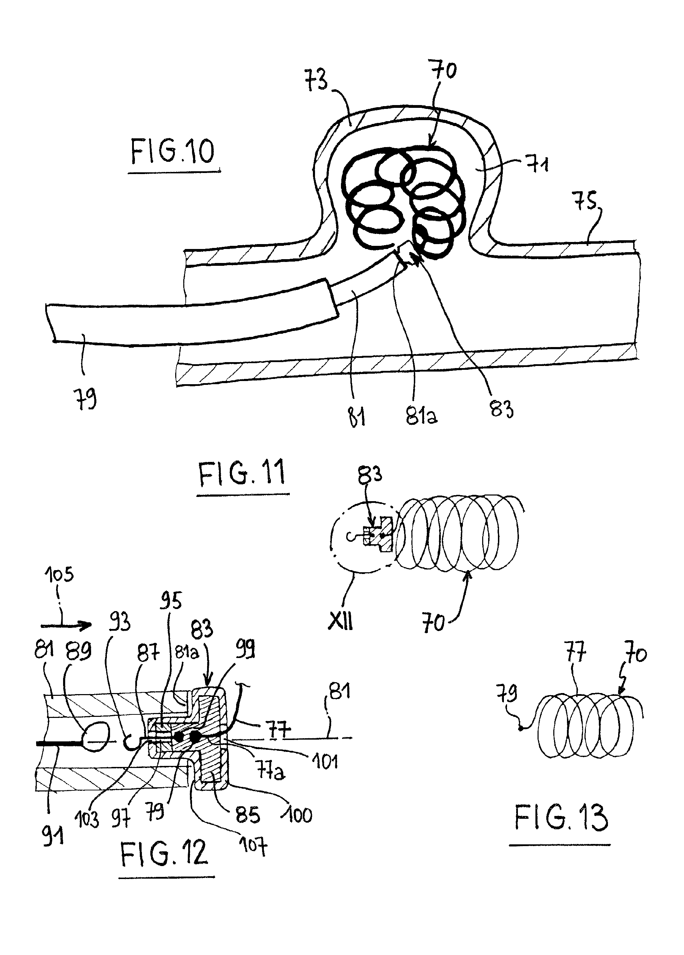

Sur les figures 10 à 13, c'est le cas de l'implantation d'un moyen d'embolisation 70 qui est illustré , consistant en l'espèce en un enroulement filamentaire (typiquement dénommé "embolic coil" ou "embolic coil means").

Cet enroulement filamentaire est destiné à venir remplir une partie au moins de la cavité 71 créée par un anévrisme 73 sur un vaisseau sanguin 75.

Typiquement, l'enroulement filamentaire 70 est constitué par un fil métallique de qualité ressort 77 enroulé en serpentin, comme on peut le voir sur la figure 13. Pour tout détail complémentaire, on se reportera à la description des colonnes 3 à 6 de US-A-5 108 407.

L'introduction vasculaire de l'enroulement filamentaire 70 est assurée par l'intermédiaire d'une gaine extérieure flexible 79 à l'intérieur de laquelle coulisse librement un poussoir tubulaire 81 agissant à son extrémité distale 81a sur le moyen de retenue libérable 83 auquel est mécaniquement lié l'enroulement 70 pendant toute la phase d'implantation jusqu'à l'anévrisme 73, voire pendant une certaine période ultérieure.

Comme illustré en vue agrandie sur la figure 12, le moyen de retenue libérable 83 s'inspire largement de la réalisation de la figure 3.

Ainsi, il comprend un bloc 85 de matériau plus malléable que celui qui constitue le filament 77 dont l'extrémité 77a terminée par la tête renflée 79 est noyée dans le bloc 85. A noter l'orientation suivant l'axe des efforts de poussée/traction de la partie terminale 77a du filament 77 (axe 81).

En regard de cette partie terminale, on trouve une tige 87 également engagée dans le bloc 85 et s'étendant suivant l'axe 81, en direction opposée (vers la partie proximale des moyens d'introduction), de manière à pouvoir être, le moment venu, saisie par un lasso 89 monté au bout d'un guide allongé 91, par l'intermédiaire d'un crochet 93 qui termine la tige 87.

Un anneau 95 complète le moyen de retenue 83, avec un orifice axial intérieur 97 dont le diamètre est inférieur à celui de la tête élargie 99 de la tige 87.

Un capuchon 100 en une matière plus dure que le bloc 85 entoure ce bloc et l'anneau 95, tout en présentant deux orifices co-axiaux 101, 103, à travers lesquels passent respectivement le filament 77 et la tige 87 de sorte que le crochet 93 est situé à l'extérieur du capuchon.

La libération du dispositif thérapeutique constitué soit par le seul enroulement filamentaire 70 (comme sur la figure 13), soit par cet enroulement avec une partie du moyen de retenue 83, s'opère comme suit.

Alors que la gaine 79 a permis d'amener jusqu'au site d'implantation (anévrisme 73) l'enroulement filamentaire 70 qui est alors positionné à l'intérieur de la cavité 71 (comme sur la figure 10), le praticien pousse axialement sur le poussoir 81 (flèche 105 sur la figure 12) tandis qu'il tire sur la tige 87, après avoir saisi son crochet 93 par l'intermédiaire du lasso 89 glissé préalablement à l'intérieur de la lumière du poussoir 81. Il applique ainsi la surface d'appui 81a du poussoir contre l'épaulement 107 du moyen de retenue 83.

Dans cette situation, l'extrémité 77a du filament 77 doit se désolidariser du bloc 85 et s'en extraire par l'orifice 101, de sorte que c'est la structure filamentaire 70 de la figure 13 qui se trouve alors libérée dans la cavité 71.

En cas de difficulté, le praticien peut continuer à pousser sur le poussoir 81 tout en tirant sur le guide du lasso 89, dès lors que ce surcroît d'effort doit entraîner la sortie forcée de la tête 99 à travers l'orifice de diamètre restreint 97 et, dans son prolongement, à travers l'orifice 103, entraînant ainsi avec le lasso la tige 87 et laissant l'enroulement 70 engagé en 77a à l'intérieur du capuchon 100, à la manière de ce qui s'est passé en relation avec la figure 3 lorsque seul l'effort F3 a permis d'extraire le crochet 11 hors de l'élément de retenue 13.

A noter qu'on pourrait choisir d'adapter les solutions des figures 5 et 6 au cas de la figure 12.

A noter également qu'une inversion de structure aurait pu être prévue notamment dans le mode de réalisation des figures 7 à 9, avec un bloc en matériau malléable lié au dispositif 40 et un ou plusieurs "éléments allongés durs" appartenant au tube de guidage 51.

**Revendications**

1. Appareil médical intraluminal adapté pour être disposé dans un conduit anatomique, l'appareil comprenant :

   - une première partie (11, 29 ; 53 ; 87) adaptée pour être retirée du conduit après y avoir été placée pendant une première période,

- et une seconde partie (1, 40, 70) liée à la première partie pendant la première période, par l'intermédiaire d'un moyen de retenue libérable (13, 53, 83), cette seconde partie étant donc placée avec la première partie et le moyen de retenue libérable dans le conduit (5, 75), pendant la première période, cette seconde partie étant constituée par, ou comprenant, un dispositif thérapeutique (1, 40, 70) adapté pour demeurer dans le conduit après la première période,

l'appareil étant **caractérisé en ce que**, pour assurer une séparation entre la première et la deuxième parties et permettre à la deuxième partie (1, 40, 70) de demeurer dans le conduit à l'issue de la première période, le moyen de retenue libérable (13, 53, 83) présente une première liaison séparable qui se rompt si une première force (F2) de séparation est exercée entre la première et la deuxième parties à l'endroit du moyen de retenue libérable, alors que la seconde partie est dans un état de retenue, fixée au conduit (5) ou en appui contre une surface d'appui (53a, 81a) d'un élément complémentaire (53, 81) de l'appareil, cet élément complémentaire étant indépendant et monté mobile vis-à-vis des première et seconde parties, pour être retiré hors du conduit le moment venu.

2. Appareil selon la revendication 1, **caractérisé en ce que**, pour constituer ladite première liaison séparable, au moins un élément allongé dur (15, 43a, 43b, 77a) est associé avec le moyen de retenue libérable et est lié de façon permanente à la première partie ou à la seconde partie, ce moyen de retenue libérable comprenant un bloc (23, 57, 85) de matériau plus malléable que la matière de l'élément allongé dur, lequel élément allongé dur est noyé à l'intérieur du bloc de matériau malléable pendant la première période et en est séparé à force lorsque ladite première force (F2) de séparation est exercée entre la première et la seconde parties.

3. Appareil selon la revendication 2, **caractérisé en ce que** :

   - la matière du bloc de matériau malléable (23, 57, 85) est, ou comprend, une silicone,
   - et l'élément allongé dur est en un matériau parmi un métal, un alliage métallique ou une matière plastique plus dure que la silicone.

4. Appareil selon l'une des revendications 2 à 4, **caractérisé en ce que** le(les) élément(s) allongé(s) dur(s) (15, 43a, 43b, 77) se termine(nt) par une zone de retenue renforcée présentant une section élargie ou une partie terminale courbée.

5. Appareil médical selon l'une quelconque des revendications précédentes, **caractérisé en ce que** l'élément de retenue libérable présente en outre une seconde liaison séparable (59 ; 11a, 29) existant, à l'issue de ladite première période, entre l'élément de retenue libérable et des moyens d'accouplement (11',11", 51) retirables du conduit à l'issue de la première période et adaptés pour se séparer de l'élément de retenue libérable si une seconde force prédéterminée (F3) de séparation est exercée entre eux, cette seconde force étant supérieure à la première, en valeur nominale.

6. Appareil selon la revendication 5, **caractérisé en ce que** la séparation des moyens d'accouplement (11") vis-à-vis de l'élément de retenue (13") s'opère lorsque la première force (F2) devient supérieure à la seconde force (F3), dans des conditions particulières, par passage forcé d'une partie (11a, 11"b) des moyens d'accouplement à travers un orifice calibré (31) dans lequel ces moyens d'accouplement sont retenus jusqu'à ce que ladite seconde force soit exercée.

7. Appareil médical selon l'une quelconque des revendications précédentes, **caractérisée en ce que** :

   - il s'agit d'un dispositif de filtration sanguine, pouvant être adapté pour traiter une embolie, le dispositif comprenant un filtre sanguin qui présente un axe (19) et qui comprend :

     . une pluralité d'éléments filtrants intraluminaux (15), les éléments étant agencés pour être disposés dans une configuration de filtration dans la lumière (3) du vaisseau, ces éléments filtrants présentant une zone (15b) à l'endroit de laquelle ils sont radialement rapprochés les uns des autres dans cette configuration de filtration,
     . et un élément de jonction (17) relié à la pluralité des éléments filtrants (15) et qui, au moins à la libération de l'élément de retenue, joint entre eux les éléments filtrants alors disposés dans une configuration ouverte où ces éléments sont radialement écartés les uns des autres,

   - le moyen (13, 13', 13") de retenue libérable joignant les éléments filtrants entre eux dans la configuration de filtration, et
   - la première liaison séparable (15b, 23) existe uniquement dans la configuration de filtration, entre la zone (15b) de rapprochement radial des éléments filtrants (15) et l'élément de retenue, cette première liaison se rompant si ladite première force (F2) de séparation est exercée entre les éléments filtrants et l'élément de retenue.

8. Appareil selon la revendication 7, **caractérisé en ce que** l'élément de retenue libérable présente une seconde liaison séparable (11b-23, 11'b-33) existant, au moins dans ladite configuration de filtration, entre l'élément de retenue (13,13',13") et des moyens d'accouplement (11, 33, 7) adaptés pour se séparer de l'élément de retenue si une seconde force prédéterminée (F3) de séparation est exercée entre eux, cette seconde force étant supérieure à la première, en valeur nominale.

9. Appareil selon la revendication 8, **caractérisé en ce que** :

- les moyens d'accouplement comprennent une partie (11b, 11c) fixée à l'élément de retenue libérable (13, 13"), dans la configuration de filtration,
- et ledit élément de retenue libérable comprend :

  - un bloc (23) de matériau dans lequel est noyée la zone (15b) des éléments filtrants où ceux-ci sont radialement rapprochés les uns des autres dans la configuration de filtration,
  - un capuchon (25) renfermant le bloc et vis-à-vis duquel sont retenus ce bloc et le moyen d'accouplement (11,11"),
  - le bloc et ladite zone (15b) des éléments filtrants présentant entre eux une force nominale de séparation égale à ladite première force (F2), tandis que le capuchon et le moyen d'accouplement (11, 11") présentent entre eux une force nominale de séparation égale à ladite deuxième force (F3).

10. Appareil selon la revendication 9, **caractérisé en ce que** le bloc (23) de matériau présente une matière plus malléable que le capuchon (21) et le moyen d'accouplement (11,11").

11. Appareil selon la revendication 9 ou la revendication 10, **caractérisé en ce que** le moyen d'accouplement (11) est retenu, en cas d'efforts de traction axiale, vis-à-vis du capuchon, par l'intermédiaire d'une rondelle de sécurité (29) de laquelle le moyen d'accouplement est séparable, la force de séparation entre le moyen d'accouplement et la rondelle étant supérieure à celle qui retient les éléments filtrants (15) dans le bloc (23), en valeur nominale.

12. Appareil selon la revendication 9, **caractérisé en ce que** le moyen d'accouplement (11) est disposé à force dans un orifice (31) calibré de la rondelle.

13. Appareil selon l'une quelconque des revendications 9 à 12, **caractérisé en ce que** :

- le capuchon (21) présente, parallèlement à l'axe (19), deux ouvertures (21a, 21b) opposées à travers lesquelles passent librement respectivement une base (11b) de maintien du moyen d'accouplement (11, 11") et ladite zone (15b) des éléments filtrants, dans la configuration de filtration,
- les deux ouvertures sont entourées chacune d'un épaulement (25a, 25b) du capuchon qui participent à la retenue et du bloc (23) de matériau à l'intérieur dudit capuchon,
- la base du moyen d'accouplement et ladite zone des éléments filtrants s'étendent essentiellement parallèlement à l'axe (19) du filtre.

FIG_1

FIG_2

FIG_3

FIG_4

FIG_5

FIG_6

FIG.7

FIG.8

FIG.9

FIG.10

FIG. 11

FIG.12

FIG.13

**Office européen des brevets**

**RAPPORT DE RECHERCHE EUROPEENNE**

Numéro de la demande

EP 02 29 1131

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 00 66031 A (CONNELL PAUL T O) 9 novembre 2000 (2000-11-09) * page 21, ligne 16 - page 22, ligne 6; figures 32,33 * | 1,7 | A61F2/01 |
| D,A | US 5 383 887 A (NADAL GUY) 24 janvier 1995 (1995-01-24) | | |
| A | US 6 217 600 B1 (DIMATTEO KRISTIAN) 17 avril 2001 (2001-04-17) | | |
| A | GB 2 276 325 A (SCIMED LIFE SYSTEMS INC) 28 septembre 1994 (1994-09-28) * figures 6,7 * | 1 | |
| A | EP 0 678 284 A (BRAUN CELSA SA) 25 octobre 1995 (1995-10-25) | | |

DOMAINES TECHNIQUES RECHERCHES (Int.Cl.7)

A61F

Le présent rapport a été établi pour toutes les revendications

| Lieu de la recherche | Date d'achèvement de la recherche | Examinateur |
|---|---|---|
| LA HAYE | 7 août 2002 | Wolf, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**   EP 02 29 1131

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-08-2002

| Document brevet cité au rapport de recherche | | Date de publication | Membre(s) de la famille de brevet(s) | | Date de publication |
|---|---|---|---|---|---|
| WO 0066031 | A | 09-11-2000 | US | 6267776 B1 | 31-07-2001 |
| | | | AU | 4816400 A | 17-11-2000 |
| | | | EP | 1175185 A1 | 30-01-2002 |
| | | | WO | 0066031 A1 | 09-11-2000 |
| US 5383887 | A | 24-01-1995 | FR | 2699809 A1 | 01-07-1994 |
| | | | CA | 2112023 A1 | 29-06-1994 |
| | | | DE | 69327781 D1 | 09-03-2000 |
| | | | DE | 69327781 T2 | 31-08-2000 |
| | | | EP | 0605276 A1 | 06-07-1994 |
| | | | ES | 2143496 T3 | 16-05-2000 |
| US 6217600 | B1 | 17-04-2001 | AU | 3109301 A | 07-08-2001 |
| | | | WO | 0154616 A1 | 02-08-2001 |
| | | | US | 2001011181 A1 | 02-08-2001 |
| GB 2276325 | A | 28-09-1994 | US | 5370657 A | 06-12-1994 |
| | | | CA | 2119814 A1 | 27-09-1994 |
| | | | DE | 4410256 A1 | 29-09-1994 |
| | | | FR | 2702953 A1 | 30-09-1994 |
| | | | US | 5626605 A | 06-05-1997 |
| EP 0678284 | A | 25-10-1995 | FR | 2718949 A1 | 27-10-1995 |
| | | | EP | 1195147 A1 | 10-04-2002 |
| | | | EP | 0678284 A1 | 25-10-1995 |
| | | | FR | 2718950 A1 | 27-10-1995 |
| | | | US | 6193739 B1 | 27-02-2001 |
| | | | US | 5634942 A | 03-06-1997 |
| | | | US | 5853420 A | 29-12-1998 |

EPO FORM P0460

Pour tout renseignement concernant cette annexe : voir Journal Officiel de l'Office européen des brevets, No.12/82